# FASCICULE DE BREVET EUROPEEN

(11) **EP 2 170 208 B1**
(45) Date de publication et mention de la délivrance du brevet: **01.12.2010**
(21) Numéro de dépôt: 08826864.4
(22) Date de dépôt: 22.07.2008
(51) Int. Cl.: A61C 1/08, A61C 8/00

(54) **DISPOSITIF DE POSITIONNEMENT ET D'IMMOBILISATION D'UN GUIDE CHIRURGICAL DANS LA BOUCHE D'UN PATIENT**
VORRICHTUNG UND VERFAHREN ZUR POSITIONIERUNG UND IMMOBILISIERUNG EINER CHIRURGISCHEN BOHRLEHRE IM MUND EINES PATIENTEN
DEVICE AND METHOD FOR POSITIONING AND IMMOBILISING A SURGICAL GUIDE IN A PATIENT'S MOUTH

(30) Priorité: 23.07.2007 FR 0756670
(43) Date de publication de la demande: 07.04.2010
(73) Titulaire: Hospices Civils de Lyon, 69229 Lyon Cedex 02 (FR)
(72) Inventeur: PARIS, Marion, F-69007 Lyon (FR); FORTIN, Thomas, F-38460 Sleymieu (FR)
(74) Mandataire: Blanchard, Eugène Gilles
(86) Numéro de dépôt international: PCT/FR2008/051378
(87) Numéro de publication internationale: WO 2009/016312

(56) Documents cités:
- EP-A- 0 574 868
- WO-A-00/27331
- DE-A1- 10 036 027
- DE-A1- 10 049 938
- US-A- 5 370 117
- US-A1- 2005 045 187
- US-B1- 7 024 237

## Description

La présente invention concerne le domaine technique des guides chirurgicaux utilisés pour la mise en place d'implants dentaires.

Des demandes de brevet WO94/26199 et EP0979057 ont proposé des guides chirurgicaux particulièrement adaptés à un positionnement d'une très grande précision des implants dentaires. Les guides chirurgicaux proposés par ces documents sont plus particulièrement adaptés pour une réalisation en plusieurs étapes. Une de ces étapes consiste, après réalisation du corps du guide sur la base d'une empreinte du maxillaire ou de la mandibule du patient à appareiller, à effectuer un scanner des maxillaires du patient avec le corps du guide en position. Une étape suivante consiste, sur la base des informations issues du scanner et du guide, à mettre en place dans le corps du guide des tubes de guidage du perçage au moyen d'un centre d'usinage automatisé. La fabrication du guide chirurgical ainsi terminée, ce dernier est positionné sur le maxillaire ou la mandibule du patient et immobilisé au moyen de vis d'ancrage intra-osseuses pour permettre au chirurgien de réaliser dans l'os de ce maxillaire ou mandibulaire, les forages pré-implantaires sans risque de déplacement intempestif du guide chirurgical.

De tels guides chirurgicaux et leur mode de mise en oeuvre sont pleinement adaptés pour un usage sur des patients sains dont les muqueuses n'ont pas subi de remaniement particulier. En effet, la radiothérapie ainsi que la chirurgie résectrice ou de reconstruction altère la physionomie des tissus mous et diminue le potentiel de cicatrisation. Dans le cadre des patients atteints d'un cancer des voies dérodigestives supérieures, les muqueuses sont altérées, le potentiel de cicatrisation est diminué et il existe un risque vital de créer une osthoéradionécrose consécutive à une chirurgie invasive. Il est nécessaire chez ce type de patient, de préserver la vascularisation osseuse donc le périoste et diminuer les portes d'entrées infectieuses. Il n'est donc pas possible d'envisager une immobilisation des guides chirurgicaux par un ancrage au moyen de vis intra-osseuse.

On connaît également différents dispositifs, selon le préambule de la revendication 1, tels que décrits par exemple dans les documents DE 100 49 938, DE 100 36 027 ou encore EP 0 574 868 A2 permettant le positionnement et/ou le calcul de paramètres physiologiques en vue de la préparation et la mise en place d'implants dentaires et qui comportent différents systèmes rigides de stabilisation sur la tête d'un patient. Cependant, ces dispositifs, outre leur caractère malpratique pour le chirurgien, ne permettent pas bien souvent d'obtenir une réelle stabilité des guides chirurgicaux et/ou moyens de mesures sur le patient. Par ailleurs, ces dispositifs ne sont pas plus adaptés que les guides chirurgicaux préalablement évoqués au traitement de patients atteint de cancer des voies dérodigestives supérieures.

Il est donc apparu le besoin de disposer de moyens permettant d'utiliser les guides chirurgicaux sur de tels patients en diminuant l'invasivité du geste chirurgical au niveau des zones d'implantation et en conservant la grande précision d'implantation offerte par la mise en oeuvre des guides chirurgicaux.

Afin d'atteindre ces objectifs, l'invention concerne un dispositif de positionnement et d'immobilisation sur un patient d'un guide chirurgical mandibulaire ou maxillaire qui est adapté à la préparation de la pose au moins d'un implant dentaire et qui comprend :
- un corps formant un masque comprenant une face d'appui adaptée pour emboîter une partie au moins du visage dans une position ouverte de la bouche du patient,
- des moyens de fixation d'un guide chirurgical au corps, et
- un organe de liaison coopérant avec les moyens de fixation et le guide chirurgical pour immobiliser le guide chirurgical sur la mandibule ou le maxillaire du patient bouche ouverte et s'étendant en extension du guide chirurgical à l'extérieur de la cavité buccale lorsque le guide chirurgical est placé en relation avec la mandibule ou le maxillaire du patient.

La mise en oeuvre d'un tel dispositif de positionnement et d'immobilisation permet, grâce au corps, d'avoir un appui stable sur la tête du patient qui assure, compte tenu de la liaison entre le guide chirurgical et le corps, un positionnement et une immobilisation du guide chirurgical en position sur la mandibule ou le maxillaire du patient sans qu'il soit nécessaire de recourir à des vis d'immobilisation engagées dans la gencive et en appui contre l'os. La congruence entre la face d'appui et une partie au moins du visage offre une grande précision du positionnement ainsi qu'une très bonne immobilisation du dispositif. Le dispositif de positionnement et d'immobilisation selon l'invention permet donc de réduire les lésions muqueuses et osseuses aux seuls forages réalisés pour la mise en place des implants. Il s'agit de réaliser une chirurgie minimalement invasive.

Selon une caractéristique de l'invention permettant d'optimiser la fiabilité du positionnement, les moyens de fixation sont adaptés pour assurer une liaison rigide entre le corps et le guide chirurgical.

Dans le même sens et selon une autre caractéristique de l'invention, les moyens de fixation sont adaptés pour assurer une liaison rigide démontable entre le corps et le guide chirurgical. Le caractère démontable de cette liaison facilite la mise en place de l'ensemble en autorisant tout d'abord un placement du corps du dispositif de positionnement et d'immobilisation, puis l'adaptation du guide chirurgical en position sur la mandibule ou le maxillaire du patient.

Selon l'invention, les moyens de fixation du dispositif de positionnement destinés à coopérer avec l'organe de liaison du guide chirurgical peuvent être réalisés de toute manière appropriée et aptes à garantir une précision de positionnement satisfaisant. Selon une caractéristique de l'invention visant à permettre un assemblage aisé entre le guide chirurgical et le dispositif de positionnement et d'immobilisation, les moyens de fixation comprennent un logement de réception de l'organe de liaison qui possède une forme complémentaire de celle dudit organe de liaison, lequel s'étend en extension du guide chirurgical à l'extérieur de la cavité buccale.

Selon une forme préférée mais non strictement nécessaire de réalisation, l'organe de liaison et adapté pour s'emboîter dans le logement de réception

De plus, l'organe de liaison peut être solidaire ou amovible du guide chirurgical.

Afin d'optimiser l'efficacité de l'immobilisation, la face d'appui est, de préférence mais non nécessairement, adaptée pour emboîter une partie au moins du nez, du front, des pommettes, du menton et les angles mandibulaires du patient. En emboîtant ces régions qui présentent un relief accentué, la face d'appui contribue à une immobilisation efficace du corps en exerçant une pression bien répartie sur la surface du visage, ce qui contribue au confort du patient et réduit la gêne occasionnée par la mise en place du dispositif de positionnement et d'immobilisation selon l'invention.

Toujours dans le même sens et selon une autre caractéristique de l'invention, la face d'appui est adaptée pour emboîter, dans une position ouverte de la bouche du patient, l'intégralité du visage jusqu'à sensiblement la naissance du cuir chevelu ainsi que le menton et les angles goniaques.

Selon une caractéristique de l'invention visant à augmenter le confort du patient, le corps possède des ouvertures oculaires et bucco-nasales destinées à être placées en relation avec les yeux du patient, la bouche et les narines du patient.

Selon une forme de réalisation préférée, afin d'augmenter la stabilité du dispositif de positionnement et d'immobilisation selon l'invention, le corps forme un masque emboîtant une partie au moins du visage du patient.

Selon encore une autre caractéristique de l'invention, le dispositif de positionnement comprend des moyens de serrage destinés à venir en appui contre la partie arrière de la tête du patient pour plaquer les moyens d'appui contre le visage du patient. De tels moyens de serrage peuvent être réalisés de toute façon appropriée et, par exemple, constitués par un bandeau qui passe derrière la tête et dont les extrémités sont fixées au corps du dispositif de positionnement et d'immobilisation.

Selon une forme préférée de réalisation du dispositif de positionnement et d'immobilisation, le corps est réalisé dans un matériau thermoplastique dont la température de ramollissement est comprise entre 38°C et 80°C, et, de préférence, entre 40°C et 50°C. Cette forme préférée de réalisation est particulièrement adaptée pour une fabrication du corps du dispositif par un moulage directement sur le visage du patient.

L'invention concerne également un ensemble ou un kit pour la préparation d'au moins un dispositif de positionnement et d'immobilisation selon l'invention par moulage du corps du dispositif directement sur la tête du patient. Un tel ensemble ou kit comprend alors :
- au moins une plaque en un matériau thermoplastique dont la température de ramollissement est comprise entre 38°C et 80°C et, de préférence, entre 40°C et 50°C qui est destinée à former le corps du dispositif de positionnement,
- un organe de liaison d'un guide chirurgical sur le corps du dispositif de positionnement une fois la plaque mise en forme,
- et des moyens de fixation destinés à coopérer avec les moyens de liaison du guide chirurgical.

Selon une caractéristique de l'invention, la plaque comprend au moins deux fenêtres oculaires prédécoupées destinées à être placées en relation avec les yeux du patient et une fenêtre bucco-nasale prédécoupée destinée à être placée en relation avec la bouche et les narines du patient. Dans une forme préférée de réalisation, la plaque présentera alors une forme sensiblement ovale.

L'invention concerne également un guide ancillaire pour la pose d'au moins un implant dentaire chez un patient, comprenant :
■ un guide chirurgical mandibulaire ou maxillaire,
■ un dispositif, selon l'une des revendications 1 à 11, de positionnement et d'immobilisation sur le patient du guide chirurgical.

Selon une caractéristique de l'ancillaire selon l'invention, le guide chirurgical et l'organe de liaison du dispositif de positionnement sont solidaires ou amovibles l'un de l'autre.

Toujours selon l'invention, l'ancillaire proposé est de préférence tel que l'organe de liaison et les moyens de fixation du dispositif de positionnement coopèrent l'un et l'autre de façon amovible.

L'invention concerne aussi un procédé de fabrication d'un dispositif de positionnement et d'immobilisation selon l'invention qui comprend une étape de moulage du corps du dispositif à la forme du visage du patient en position ouverte de la bouche.

Bien entendu, les différentes caractéristiques de l'invention évoquées ci-dessus peuvent être mises en oeuvre les unes avec les autres selon différentes combinaisons lorsqu'elles ne sont pas incompatibles ou exclusives les unes des autres.

Par ailleurs, diverses autres caractéristiques de l'invention ressortent de la description faite ci-dessous en référence aux dessins annexés qui montrent, à titre d'exemples non limitatifs, une forme de réalisation de l'objet de l'invention.

La **fig. 1** est une vue schématique du dispositif de positionnement et d'immobilisation selon l'invention placé sur la tête d'un patient.

La **fig. 2** est une vue schématique d'un guide chirurgical, avant mise en place de tubes de guidage, destiné à être associé au dispositif de positionnement d'immobilisation tel qu'illustré à la **fig. 1****.**

La **fig. 3** est une vue schématique du guide chirurgical tel qu'illustré à la **fig. 2****,** après mise en place de tubes de guidage.

La **fig. 4** est une vue schématique de la mise en oeuvre, dans le cadre d'une intervention chirurgicale d'implantation, du dispositif de positionnement et d'immobilisation, illustré **fig. 1****,** en association avec le guide chirurgical illustré **fig. 3****.**

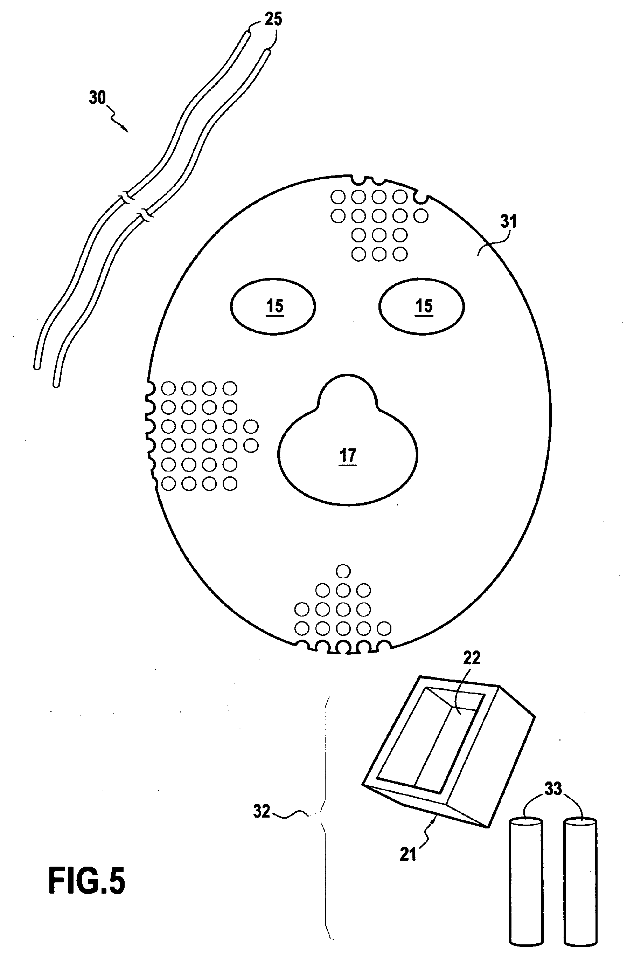

La **fig. 5** est une vue schématique d'un ensemble d'éléments ou d'un kit destinés à permettre la réalisation du dispositif de positionnement et d'immobilisation tel qu'illustré **fig. 1****.**

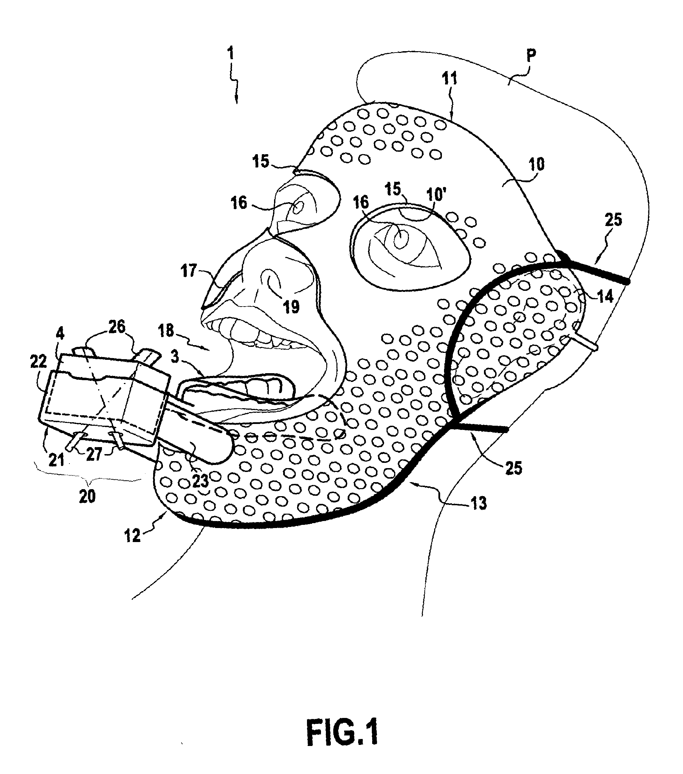

La présente invention vise à proposer un dispositif de positionnement et d'immobilisation, tel qu'illustré à la **figure 1** et désigné dans son ensemble par la référence **1,** destiné à faciliter la pose d'implants dentaires conçus pour assurer le maintien d'une prothèse dentaire.

La problématique de la pose d'implants dentaires réside dans le positionnement précis des implants dans des régions du maxillaire ou de la mandibule offrant un capital osseux suffisant pour garantir une stabilité de l'implant et une pérennité de son ancrage. Afin d'assurer un positionnement optimal, il est mis en oeuvre un processus de détermination d'une position des implants et de la mise en place, tel que par exemple décrit dans les demandes WO 94/26 199 et EP 979 057.

Une première étape consiste à réaliser une empreinte de la mâchoire à appareiller grâce à un matériau d'empreinte tel que du silicone, un alginate, un hydrocoloïde ou tout autre matériau prévu à cet effet. Il est alors réalisé à partir de cette empreinte un contre-moulage ou modèle en plâtre de la mâchoire à appareiller.

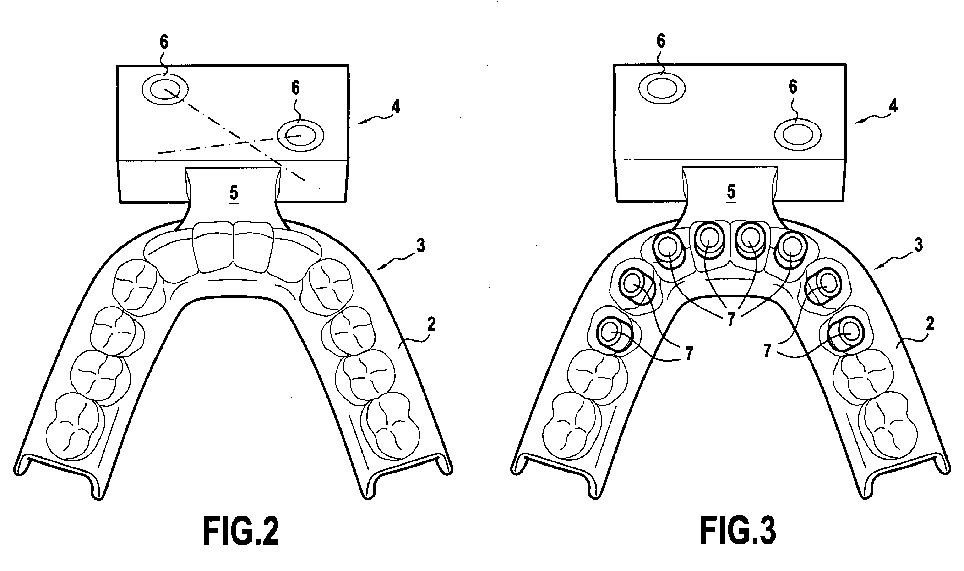

Une deuxième étape consiste à réaliser à partir du modèle en plâtre une prothèse d'étude qui est positionnée dans la bouche du patient, de manière à vérifier sa parfaite adaptation à la morphologie du patient. Une fois la forme de la prothèse d'étude finalisée et validée, il est réalisé, à partir de cette prothèse d'étude, le corps en résine **2** d'un guide chirurgical **3** tel qu'illustré à la **fig.2**. Afin de permettre une visualisation au scanner du corps du guide chirurgical dans le cadre d'une étape à venir, il sera utilisé pour la fabrication du corps du guide chirurgical une résine à radio opaque telle que la résine commercialisée sous la dénomination SR Vivo TAC/SR Ortho TAC par la société Ivoclar Vivadent sise à Saint Jorioz en France. Conformément à une procédure connue et pour autoriser une prise en charge ultérieure du guide chirurgical par un robot d'usinage, des moyens de liaison **4** sont fixés au corps **2** de manière à se trouver à l'extérieur de la cavité buccale lorsque le guide chirurgical **3** est placé à l'intérieur de cette dernière. Selon l'exemple illustré, il est utilisé en tant que moyen de liaison **4** un pavé préfabriqué en résine acrylique tel que par exemple commercialisé sous la dénomination X-cube par la société KEYSTONE Inc. sise à Burlington, Massachusetts, USA. Le pavé **4** est alors lié au corps **2** par un bras en résine **5,** de manière à laisser suffisamment d'espace pour la lèvre entre le corps **2** et le pavé. Ainsi, la longueur du bras **5,** mesurée entre le pavé **4** et le corps **2,** possède une valeur comprise entre 25 mm et 35 mm. Conformément à une caractéristique connue du pavé X-cube, ce dernier comprend deux tubes **6** en matériau radio opaque tel que du titane dont les axes sont situés dans deux plans parallèles et sont orientés à **90°** l'un de l'autre. Les deux tubes **6** sont prévus pour, d'une part, permettre un repérage du pavé de liaison **4** au scanner et, d'autre part, autoriser une fixation rigide du guide chirurgical sur le banc d'un robot d'usinage. Ainsi, le pavé de liaison **4** et ses tubes **6** forment un marqueur fiduciaire. Bien entendu, tout moyen de liaison adapté autre que le X-cuve pourrait être utilisé.

Selon cet exemple, la prothèse d'étude et le guide chirurgical sont conçus pour un appareillage de la mandibule d'un patient. Bien entendu, il pourrait être réalisé de la même manière une prothèse d'études et un guide chirurgical pour le maxillaire d'un patient.

L'étape suivante consiste à réaliser un examen radiologique de la mandibule du patient avec le guide chirurgical, tel que réalisé précédemment, en place pour déterminer, en fonction de l'état et de la répartition de la masse osseuse du patient, ainsi que de l'emplacement prévu des prothèses, le meilleur emplacement possible pour les implants. L'examen radiologique, réalisé généralement au moyen d'un scanner, permet une planification par ordinateur de l'implantation des implants au moyen, par exemple, du logiciel CADIMPLANT commercialisé par la société KEYSTONE. Le fichier électronique de cette planification est alors utilisé par un robot d'usinage pour placer dans le corps du guide chirurgical des tubes de guidage **7** comme le montre la **fig. 3****.** Le pavé de liaison **4,** visible lors de l'examen radiologique, est utilisé pour la fixation du guide chirurgical **3** sur le banc du robot d'usinage. Cela définit alors une référence commune à l'usinage et à la planification et permet ainsi une parfaite coïncidence entre les axes des implants selon l'implantation planifiée et les axes des tubes de guidage **5**.

Une fois le guide d'usinage **3** pourvu de ses tubes **5** ainsi fabriqués, il est replacé dans la bouche du patient et, selon l'exemple illustré, au niveau de la mandibule de ce dernier. Le chirurgien pourra alors réaliser des forages pré-implantaires en suivant les tubes **7.** Afin de conserver tout le bénéfice de la précision obtenue par la même mise en oeuvre du guide chirurgical **3** avec ces moyens de liaison **4,** de l'examen radiologique, de la planification par ordinateur et du robot d'usinage, il est nécessaire que le guide chirurgical soit, après son usinage, repositionné très précisément à l'emplacement qu'il occupait lors de l'examen radiologique, de manière qu'en suivant les guides de perçage, le chirurgien ait l'assurance de réaliser des orifices d'implantation conformes à la planification.

Afin d'atteindre cet objectif de précision, l'invention propose de mettre en oeuvre le dispositif de positionnement et d'immobilisation **1,** d'une part, lors de l'examen radiologique et, d'autre part, lors de l'intervention chirurgicale, de manière à offrir une référence de positionnement du guide chirurgical commune à ces deux étapes du processus de mise en place des implants.

À cet effet, et comme le montre plus particulièrement la **fig.1**, le dispositif de positionnement et d'immobilisation **1** comprend un corps **10** qui vient en appui sur la tête du patient **P.** Selon l'exemple illustré, le corps **10** comprend, en tant que moyens d'appui sur l'extérieur de la tête du patient, une face d'appui **10'** qui est située sous le corps **10** et qui possède une forme complémentaire de la forme du visage du patient **P.** Selon cet exemple, le corps **10** est plus particulièrement réalisé sous la forme d'un masque qui emboîte quasiment tout le visage du patient **P** en s'étendant depuis la naissance **11** du cuir chevelu jusqu'au menton **12** et aux angles goniaques **13** ou aux angles de la mandibule. Il sera remarqué que le corps **10** emboîte également les oreilles **14** du patient **P,** bien qu'il puisse également être envisagé que le corps **10** possède des échancrures au niveau des oreilles **14,** de manière à laisser ces dernières dégagées. Afin d'assurer un certain confort au patient **P,** le corps **10** présente deux fenêtres oculaires **15** placées en relation avec les yeux **16** du patient **P.** Le corps **10** comprend également une fenêtre bucco-nasale **17,** placée en relation avec la bouche **18** et les narines **19** afin de permettre au patient de respirer normalement par ses narines. Il sera remarqué que le corps **10** est adapté pour emboîter le visage du patient dans une position dans laquelle la bouche **18** est suffisamment ouverte pour permettre au chirurgien de travailler à l'intérieur de celle-ci, le corps **10** étant en position, notamment, de permettre une introduction et un retrait du guide chirurgical **3**.

Afin de permettre un assemblage entre le corps **10** et le guide chirurgical **3,** le dispositif de positionnement et d'immobilisation **1** comprend, en outre, des moyens de fixation **20** destinés à coopérer avec les moyens de liaison **4** du guide chirurgical **3.** Selon l'exemple illustré, les moyens de fixation **20** sont adaptés pour assurer une liaison rigide entre le corps **10** et le guide chirurgical **3.** À cet effet, les moyens de fixation **20** comprennent un boitier **21** qui délimite un logement de réception **22** de forme complémentaire de celles des moyens de liaison **4** et donc, selon l'exemple illustré, de forme sensiblement parallélépipédique. Le logement de réception **22** est ouvert sur le dessus de manière à permettre un engagement ou emboîtement du pavé constitutif des moyens de liaison **4.** Le boîtier **21** est en outre lié au corps **10** par un ou plusieurs bras rigides **23.**

Le dispositif de positionnement et d'immobilisation **1** selon l'invention tel qu'ainsi constitué est mis en oeuvre, dans le cadre du processus décrit précédemment, de la manière suivante.

Préalablement à l'examen radiologique, le dispositif de positionnement et d'immobilisation **1** est placé sur le visage du patient **P** en étant parfaitement plaqué par des moyens de serrage **25,** tels que par exemple des liens souples élastiques ou non liés au corps **10** et passant à l'arrière du crâne du patient comme le montre la **fig. 1****.** Ensuite, le guide chirurgical ou son ébauche tels qu'illustrés à la **fig.2** sont mis en place en relation avec la mandibule du patient. Les moyens de liaison **4** coopèrent alors avec les moyens de fixation **20** en étant engagés dans le logement **22** du boîtier **21.** Afin d'éviter tout mouvement intempestif des moyens de liaison **4,** des tiges de verrouillage **26** sont engagées dans les tubes **6** jusqu'à traverser des alésages de verrouillage **27** aménagés dans le fond du boîtier **21.**

Le patient ainsi équipé peut alors bénéficier de l'examen radiologique nécessaire à la planification de l'implantation. À l'issue de cet examen radiologique, le guide chirurgical **3** est retiré et le dispositif de positionnement et d'immobilisation **1** est enlevé du visage du patient.

Ensuite, la planification de l'implantation et l'usinage du guide chirurgical **3** sont effectués comme expliqué précédemment.

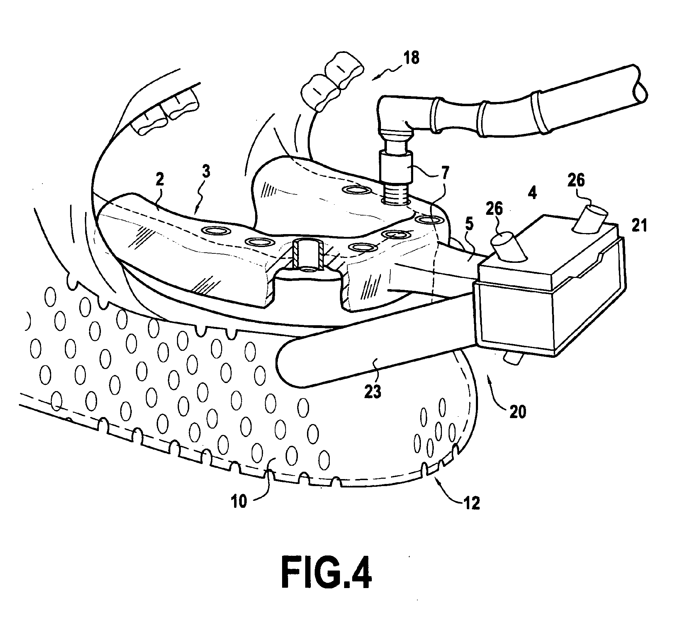

Pour la phase d'implantation, le dispositif de positionnement et d'immobilisation **1** est de nouveau placé sur le visage du patient. Le guide chirurgical **3** tel qu'illustré à la **fig. 3** est de nouveau placé dans la bouche du patient en relation avec la mandibule de ce dernier, les moyens de liaison **4** étant engagés dans les moyens de fixation en y étant verrouillés par les tiges **26.** Le chirurgien peut alors procéder aux perçages en étant guidé par les tubes **7** comme le montre la **fig. 4** dans le cadre d'une intervention sous anesthésie générale par exemple.

La mise en oeuvre du dispositif d'immobilisation et de fixation **1** selon l'invention permet donc de garantir une très bonne concordance entre la position du guide chirurgical lors de l'examen radiologique et la position de ce même guide chirurgical lors du geste chirurgical en lui-même.

Selon l'exemple illustré, le verrouillage des moyens de liaison **4** avec les moyens de fixation **20** est effectué avec les tiges **26,** toutefois, tout autre moyen de verrouillage permettant d'éviter tout mouvement relatif entre le moyen de liaison et les moyens de fixation pourraient être envisagés.

Le dispositif de positionnement et d'immobilisation **1** selon l'invention peut être fabriqué de différentes façons.

Dans une forme préférée de mise en oeuvre, le dispositif **1** est fabriqué sur mesure pour chaque patient. A cet effet, il est utilisé un ensemble ou kit, tel qu'illustré à la **fig. 4** et désigné dans son ensemble par la référence **30,** qui comprend une plaque **31** en un matériau thermoplastique dont la température de ramollissement est comprise entre 38°C et 80°C et, de préférence, entre 40°C et 50°C. La plaque **30** est, par exemple, réalisée en un matériau thermoplastique à basse température commercialisé sous la dénomination Posicast-lite par la société Simmed BV sise à Reeuwijk aux Pays-Bas. La plaque **31** qui présente, selon l'exemple illustré, une forme sensiblement ovale et qui est destinée à former le corps du dispositif de positionnement **1,** possède deux fenêtres oculaires **15** prédécoupées ainsi qu'une fenêtre bucco-nasale **17** également prédécoupée. L'ensemble **30** comprend aussi des éléments **32** pour constituer les moyens de fixation **20** destinés à coopérer avec les moyens de liaison **4** du guide chirurgical **3.** Selon l'exemple illustré, les éléments **32** comprennent, d'une part, le boîtier **21** qui définit le logement **22** de réception du pavé de liaison **4** et, d'autre part, deux blocs ou cylindres de résine destinés à assurer un fixation rigide du boitier **21** au corps ou masque **10,** comme cela apparaîtra par la suite. L'ensemble **30** comprend enfin des liens élastiques **25** destinés à constituer les moyens de serrage du dispositif sur la tête du patient **P.**

La fabrication du dispositif **1** est effectuée avant l'examen radiologique et après fabrication du guide chirurgical **3** ou de son ébauche, tels qu'illustrés à la **fig.2****.**

Une première étape de cette fabrication consiste à mouler la plaque **31** sur le visage du patient dans une position ouverte de la bouche. À cet effet, la plaque **31** est plongée pendant une minute environ dans un bain d'eau chaude à **70** °C. La plaque **31** est ensuite séchée entre deux serviettes qui absorbent l'eau en excès et ramènent la plaque à une température de 45 °C environ, température à laquelle la plaque **31** est dans un état ramolli ou plastique et peut être appliquée sur le visage du patient pour en épouser parfaitement les formes. Bien entendu, lors de ce moulage, la plaque **31** sera placée de manière à disposer les fenêtres oculaires **15** au niveau des yeux du patient et la fenêtre bucco-nasale en relation avec les narines et la bouche ouverte du patient. Une fois ce moulage réalisé, on laisse refroidir sur le visage du patient la plaque **31** qui forme alors le corps **10** du dispositif **1.** Il est à noter que le moulage de la plaque **31** peut avantageusement être réalisé par deux opérateurs travaillant en même temps.

Lorsque le corps **10** a refroidi et retrouvé sa rigidité, il peut être retiré du visage et faire l'objet d'opérations de finition visant notamment à supprimer les arêtes vives susceptibles de blesser le patient. Les liens élastiques **25** sont alors fixés au corps **10** qui est replacé sur le visage du patient afin de procéder à la mise en place des moyens de fixation **20.** Pour ce faire, le boîtier **21** est adapté sur les moyens de liaison **4** du guide chirurgical **3** et verrouillé par les tiges **26.** Ensuite, le guide chirurgical **3** est disposé dans la bouche du patient en relation avec la mandibule de ce dernier dans une position correspondant à la position optimale des prothèses devant équiper la mandibule. Le boîtier **21** est alors fixé rigidement sur la partie antérieure du corps **10** au moyen des cylindres **33** et/ou d'une résine adhésive, telle que par exemple de la résine commercialisée sous la dénomination Unifast Trad par la société GC France sise à Bonneuil sur Marne en France.

Après polymérisation de la résine, la fabrication du dispositif de positionnement et d'immobilisation **1** selon l'invention est achevée et il peut être procédé à l'examen radiologique. Il doit être remarqué que l'examen radiologique peut être effectué soit juste après l'achèvement de la fabrication du dispositif **1,** soit ultérieurement, dans la mesure où, pour retirer le dispositif **1** il suffit de déverrouiller la liaison entre le guide chirurgical **3** et le dispositif **1** en retirant les tiges **26** puis d'enlever le guide chirurgical **3** et enfin le dispositif **1.** Il doit être également noté que la rigidité du corps **10** permet de le remettre en place et de le retirer autant de fois que nécessaire tout en retrouvant, une fois assuré le verrouillage de la liaison entre le guide chirurgical **3** et le dispositif **1,** la même position du guide chirurgical **3** que celle occupée lors de la fabrication du dispositif de positionnement et d'immobilisation **1.** C'est cette position qui sera retrouvée pour l'examen radiologique et pour l'intervention chirurgicale une fois la fabrication du guide chirurgical **3** achevée, comme cela a été expliqué précédemment.

Bien entendu, la fabrication du dispositif de positionnement et d'immobilisation **1** selon l'invention pourrait être effectuée d'une tout autre manière dans la mesure où cette fabrication est réalisée de façon à garantir l'identité de la position du guide chirurgical **3** à chaque mise en place de l'ensemble sur le visage du patient.

Par ailleurs, selon l'exemple décrit précédemment le guide chirurgical comprend des tubes de guidage **7.** Toutefois ces tubes peuvent être remplacés chacun par un simple alésage réalisé directement dans le guide chirurgical.

Ainsi, diverses modifications peuvent être apportées à l'invention dans le cadre des revendications.

## Revendications

1. Dispositif de positionnement et d'immobilisation sur un patient d'un guide chirurgical (3) mandibulaire ou maxillaire qui est adapté à la préparation de la pose d'au moins un implant dentaire, **caractérisé en ce qu'**il comprend :
- un corps (10) formant un masque comprenant une face d'appui (10') adaptée pour emboîter une partie au moins du visage dans une position ouverte de la bouche du patient,
- des moyens de fixation (20) d'un guide chirurgical (3) au corps (10), et
- un organe de liaison (4) coopérant avec les moyens de fixation (20) et le guide chirurgical (3) pour immobiliser le guide chirurgical (3) sur la mandibule ou le maxillaire du patient bouche ouverte et s'étendant en extension du guide chirurgical (3) à l'extérieur de la cavité buccale lorsque le guide chirurgical (3) est placé en relation avec la mandibule ou le maxillaire du patient.

2. Dispositif de positionnement selon la revendication 1, **caractérisé en ce que** les moyens de fixation (20) sont adaptés pour assurer une liaison rigide entre le corps (10) et le guide chirurgical (3).

3. Dispositif de positionnement selon la revendication 1, **caractérisé en ce que** les moyens de fixation (20) sont adaptés pour assurer une liaison rigide démontable entre le corps (10) et le guide chirurgical (3).

4. Dispositif de positionnement selon l'une des revendications 1 à 3, **caractérisé en ce que** les moyens de fixation (20) comprennent un logement (22) de réception de l'organe de liaison (4) qui possède une forme complémentaire de celle dudit logement (22).

5. Dispositif de positionnement selon la revendication 4, **caractérisé en ce que** l'organe de liaison (4) et adapté pour s'emboîter dans le logement de réception (22).

6. Dispositif de positionnement selon l'une des revendications 1 à 5, **caractérisé en ce que** l'organe de liaison (4) est solidaire ou amovible du guide chirurgical (3).

7. Dispositif de positionnement selon l'une des revendications 1 à 6, **caractérisé en ce que** la face d'appui (10') est adaptée pour emboîter une partie du nez, du front, des pommettes et du menton du patient et les angles mandibulaires.

8. Dispositif de positionnement selon la revendication 7, **caractérisé en ce que** la face d'appui (10') est adaptée pour emboîter, dans une position ouverte de la bouche du patient, l'intégralité du visage jusqu'à sensiblement la naissance (11) du cuir chevelu, ainsi que le menton (12) et les angles (13) de la mandibule.

9. Dispositif de positionnement selon l'une des revendications 1 à 8 **caractérisé en ce que** le corps possède des ouvertures oculaires (15) et bucco-nasale (17) destinées à être placées en relation avec les yeux (16), la bouche (18) et les narines (19) du patient.

10. Dispositif de positionnement selon l'une des revendications 1 à 9 **caractérisé en ce qu'**il comprend des moyens de serrage (25) destinés à venir en appui contre la partie arrière de la tête du patient pour plaquer les moyens d'appui (10') contre le visage du patient.

11. Dispositif de positionnement selon l'une des revendications 1 à 10, **caractérisé en ce que** le corps (10) est réalisé dans un matériau thermoplastique dont la température de ramollissement est comprise entre 38°C et 80°C et, de préférence, entre 40°C et 50°C.

12. Ensemble pour la préparation d'au moins un dispositif de positionnement selon la revendication 11, **caractérisé en ce qu'**il comprend :
■ au moins une plaque (31) en un matériau thermoplastique dont la température de ramollissement est comprise entre 38°C et 80°C et, de préférence, entre 40°C et 50°C qui est destinée à former le corps du dispositif de positionnement,
■ un organe de liaison (4) d'un guide chirurgical (3) sur le corps du dispositif de positionnement une fois la plaque (31) mise en forme,
■ et des moyens de fixation (20) destinés à coopérer avec les moyens de liaison (4) du guide chirurgical (3).

13. Ensemble selon la revendication 12, **caractérisé en ce que** la plaque (31) comprend au moins deux fenêtres oculaires (15) prédécoupées destinées à être placée en relation avec les yeux du patient et une fenêtre bucco-nasale (17) prédécoupée destinée à être placée en relation avec la bouche et les narines du patient.

14. Ancillaire pour la pose d'au moins un implant dentaire chez un patient, comprenant :
■ un guide chirurgical (3) mandibulaire ou maxillaire,
■ un dispositif (1), selon l'une des revendications 1 à 11, de positionnement et d'immobilisation sur le patient du guide chirurgical (3).

15. Ancillaire selon la revendication 14, **caractérisé en ce que** le guide chirurgical (3) et l'organe de liaison (4) du dispositif de positionnement (1) sont solidaires ou amovibles l'un de l'autre.

16. Ancillaire selon l'une des revendications 14 ou 15, **caractérisé en ce que** l'organe de liaison (4) et les moyens de fixation (20) du dispositif de positionnement coopèrent l'un et l'autre de façon amovible.

17. Procédé de fabrication d'un dispositif de positionnement et d'immobilisation selon l'une des revendications 1 à 11, **caractérisé en ce qu'**il comprend une étape de moulage du corps du dispositif à la forme du visage du patient en position ouverte de la bouche.

## Claims

1. A positioning and immobilization device for positioning and immobilizing a mandibular or maxillary surgical guide (3) on a patient, which guide is adapted to preparing for and to fitting at least one dental implant, said positioning and immobilization device being **characterized in that** it comprises:
a body (10) forming a mask having a bearing face (10') adapted to fit over at least a portion of the patient's face with the patient being in a mouth-open position;
fastener means (20) for fastening a surgical guide (3) to the body (10); and
a link member (4) co-operating with the fastener means (20) and with the surgical guide (3) to immobilize the surgical guide (3) on the mandible or the maxilla of the patient with the mouth open, and extending from the surgical guide (3) outside the oral cavity when the surgical guide (3) is placed in register with the patient's mandible or maxilla.

2. A positioning device according to claim 1, **characterized in that** the fastener means (20) are adapted to provide a rigid link between the body (10) and the surgical guide (3).

3. A positioning device according to claim 1, **characterized in that** the fastener means (20) are adapted to provide a separable rigid link between the body (10) and the surgical guide (3).

4. A positioning device according to any one of claims 1 to 3, **characterized in that** the fastener means (20) comprise a housing (22) for receiving the link member (4) that has a shape complementary to the shape of said housing (22).

5. A positioning device according to claim 4, **characterized in that** the link member (4) is adapted to fit into the housing (22).

6. A positioning device according to any one of claims 1 to 5, **characterized in that** the link member (4) is integral with or separably secured to the surgical guide (3).

7. A positioning device according to any one of claims 1 to 6, **characterized in that** the bearing face (10') is adapted to fit over a portion of the nose, of the forehead, of the cheekbones, of the chin, and of the mandibular angles of the patient.

8. A positioning device according to claim 7, **characterized in that** the bearing face (10') is adapted to fit over the patient's entire face substantially to the hairline (11), to the chin (12), and to the angles (13) of the mandible when the patient is in a mouth-open position.

9. A positioning device according to any one of claims 1 to 8, **characterized in that** the body is provided with ocular openings (15) and with an oro-nasal opening (17), which openings are designed to be placed in register respectively with the eyes (16), and with the mouth (18) and the nostrils (19) of the patient.

10. A positioning device according to any one of claims 1 to 9, **characterized in that** it further comprises clamping means (25) designed to come to bear against the rear portion of the patient's head so as to press the bearing means (10') against the patient's face.

11. A positioning device according to any one of claims 1 to 10, **characterized in that** the body (10) is made of a thermoplastic material having a softening temperature lying in the range 38°C to 80°C, and preferably in the range 40°C to 50°C.

12. A set for preparing at least one positioning device according to claim 11, said set being **characterized in that** it comprises:
• at least one plate (31) that is made of a thermoplastic material having a softening temperature lying in the range 38°C to 80°C and, preferably, in the range 40°C to 50°C, and that is designed to form the body of the positioning device;
• a link member (4) for linking a surgical guide (3) to the body of the positioning device once the plate (31) has been shaped; and
• fastener means (20) designed to co-operate with the link means (4) of the surgical guide (3).

13. A set according to claim 12, **characterized in that** the plate (31) is provided with at least two pre-cut ocular windows (15) that are designed to be placed in register with the patient's eyes, and said plate is also provided with a pre-cut oro-nasal window (17) that is designed to be placed in register with the patient's mouth and nostrils.

14. A jig for fitting at least one dental implant in a patient, which jig comprises:
• a mandibular or maxillary surgical guide (3); and
• a device (1) according to any one of claims 1 to 11, for positioning and immobilizing the surgical guide (3) on the patient.

15. A jig according to claim 14, **characterized in that** the surgical guide (3) and the link member (4) of the positioning device (1) are integral with or separably secured to each other.

16. A jig according to claim 14 or claim 15, **characterized in that** the link member (4) and the fastener means (20) of the positioning device co-operate with each other removably.

17. A method of fabricating a positioning and immobilization device according to any one of claims 1 to 11, said method being **characterized in that** it comprises a step of molding the body of the device to the shape of the patient's face with the patient in a mouth-open position.

## Patentansprüche

1. Vorrichtung zur Positionierung und Fixierung einer chirurgischen Unterkiefer- oder Oberkiefer-Führung (3) an einem Patienten, welche für die Vorbereitung des Einsetzens mindestens eines Zahnimplantates eingerichtet ist, **dadurch gekennzeichnet, daß** sie umfaßt:
- einen Körper (10), der eine Maske bildet, die eine Anlagefläche (10') umfaßt, welche dafür eingerichtet ist, auf mindestens einen Teil des Gesichtes in einer geöffneten Position des Mundes des Patienten aufgesetzt zu werden,
- Mittel zur Befestigung (20) einer chirurgischen Führung (3) an dem Körper(10) und
- ein Verbindungsorgan (4), das mit den Befestigungsmitteln (20) und der chirurgischen Führung (3) zusammenwirkt, um die chirurgische Führung (3) am Unterkiefer oder am Oberkiefer des Patienten bei geöffnetem Mund zu fixieren, und sich in Verlängerung der chirurgischen Führung (3) außerhalb der Mundhöhle erstreckt, wenn die chirurgische Führung (3) bezüglich des Unterkiefers oder des Oberkiefers des Patienten plaziert ist.

2. Positionierungsvorrichtung nach Anspruch 1, **dadurch gekennzeichnet, daß** die Befestigungsmittel (20) dafür eingerichtet sind, eine starre Verbindung zwischen dem Körper (10) und der chirurgischen Führung (3) sicherzustellen.

3. Positionierungsvorrichtung nach Anspruch 1, **dadurch gekennzeichnet, daß** die Befestigungsmittel (20) dafür eingerichtet sind, eine lösbare starre Verbindung zwischen dem Körper (10) und der chirurgischen Führung (3) sicherzustellen.

4. Positionierungsvorrichtung nach einem der Ansprüche 1 bis 3, **dadurch gekennzeichnet, daß** die Befestigungsmittel (20) eine Aufnahme (22) für das Verbindungsorgan (4) umfassen, welches eine Form besitzt, die zu derjenigen der Aufnahme (22) komplementär ist.

5. Positionierungsvorrichtung nach Anspruch 4, **dadurch gekennzeichnet, daß** das Verbindungsorgan (4) dafür eingerichtet ist, in die Aufnahme (22) eingefügt zu werden.

6. Positionierungsvorrichtung nach einem der Ansprüche 1 bis 5, **dadurch gekennzeichnet, daß** das Verbindungsorgan (4) mit der chirurgischen Führung (3) fest verbunden oder von ihr lösbar ist.

7. Positionierungsvorrichtung nach einem der Ansprüche 1 bis 6, **dadurch gekennzeichnet, daß** die Anlagefläche (10') dafür eingerichtet ist, auf einen Teil der Nase, der Stirn, der Backenknochen und des Kinns des Patienten und die Unterkieferwinkel aufgesetzt zu werden.

8. Positionierungsvorrichtung nach Anspruch 7, **dadurch gekennzeichnet, daß** die Anlagefläche (10') dafür eingerichtet ist, in einer geöffneten Position des Mundes des Patienten auf das gesamte Gesicht des Patienten bis etwa zum Anfang (11) der Kopfschwarte sowie das Kinn (12) und die Winkel (13) des Unterkiefers aufgesetzt zu werden.

9. Positionierungsvorrichtung nach einem der Ansprüche 1 bis 8, **dadurch gekennzeichnet, daß** der Körper Augenöffnungen (15) und eine Mund- und Nasenöffnung (17) besitzt, die dazu bestimmt sind, gegenüber den Augen (16), dem Mund (18) und den Nasenlöchern (19) des Patienten angeordnet zu werden.

10. Positionierungsvorrichtung nach einem der Ansprüche 1 bis 9, **dadurch gekennzeichnet, daß** sie Spannmittel (25) umfaßt, die dazu bestimmt sind, am hinteren Teil des Kopfes des Patienten zur Anlage zu kommen, um die Anlagefläche (10') an das Gesicht des Patienten zu drücken.

11. Positionierungsvorrichtung nach einem der Ansprüche 1 bis 10, **dadurch gekennzeichnet, daß** der Körper (10) aus einem thermoplastischen Material hergestellt ist, dessen Erweichungstemperatur zwischen 38 °C und 80 °C und vorzugsweise zwischen 40 °C und 50 °C liegt.

12. Anordnung zur Herstellung mindestens einer Positionierungsvorrichtung nach Anspruch 11, **dadurch gekennzeichnet, daß** sie umfaßt:
- mindestens eine Platte (31) aus einem thermoplastischen Material, dessen Erweichungstemperatur zwischen 38 °C und 80 °C und vorzugsweise zwischen 40 °C und 50 °C liegt, welche zum Formen des Körpers der Positionierungsvorrichtung bestimmt ist,
- ein Verbindungsorgan (4) zur Verbindung einer chirurgischen Führung (3) mit dem Körper der Positionierungsvorrichtung, nachdem die Platte (31) geformt worden ist,
- und Befestigungsmittel (20), die dazu bestimmt sind, mit dem Verbindungsorgan (4) der chirurgischen Führung (3) zusammenzuwirken.

13. Anordnung nach Anspruch 12, **dadurch gekennzeichnet, daß** die Platte (31) mindestens zwei vorgeschnittene Augenfenster (15), die dazu bestimmt sind, gegenüber den Augen des Patienten angeordnet zu werden, und ein vorgeschnittenes Mund-Nasen-Fenster (17), das dazu bestimmt ist, gegenüber dem Mund und den Nasenlöchern des Patienten angeordnet zu werden, umfaßt.

14. Hilfseinrichtung für das Einsetzen mindestens eines Zahnimplantates bei einem Patienten, umfassend:
- eine chirurgische Unterkiefer- oder Oberkiefer-Führung (3),
- eine Vorrichtung (1) nach einem der Ansprüche 1 bis 11 zur Positionierung und Fixierung der chirurgischen Führung (3) an dem Patienten.

15. Hilfseinrichtung nach Anspruch 14, **dadurch gekennzeichnet, daß** die chirurgische Führung (3) und das Verbindungsorgan (4) der Positionierungsvorrichtung (1) fest miteinander verbunden oder voneinander lösbar sind.

16. Hilfseinrichtung nach einem der Ansprüche 14 oder 15, **dadurch gekennzeichnet, daß** das Verbindungsorgan (4) und die Befestigungsmittel (20) der Positionierungsvorrichtung lösbar zusammenwirken.

17. Verfahren zur Herstellung einer Vorrichtung zur Positionierung und Fixierung nach einem der Ansprüche 1 bis 11, **dadurch gekennzeichnet, daß** es einen Schritt des Formens des Körpers der Vorrichtung entsprechend der Form des Gesichtes des Patienten in einer geöffneten Position des Mundes umfaßt.
